(19) Europäisches Patentamt
European Patent Office
Office européen des brevets

(11) Veröffentlichungsnummer: **0 059 912**
**B1**

(12) # EUROPÄISCHE PATENTSCHRIFT

(45) Veröffentlichungstag der Patentschrift: **06.02.85**

(51) Int. Cl.⁴: **A 61 F 11/02**

(21) Anmeldenummer: **82101542.7**

(22) Anmeldetag: **27.02.82**

(54) **Verfahren zur Herstellung von Gehörschutz-Stopfen aus Kunststoffschaum.**

(30) Priorität: **05.03.81 DE 3108261**

(43) Veröffentlichungstag der Anmeldung: **15.09.82 Patentblatt 82/37**

(45) Bekanntmachung des Hinweises auf die Patenterteilung: **06.02.85 Patentblatt 85/6**

(84) Benannte Vertragsstaaten: **AT CH DE FR GB LI NL SE**

(56) Entgegenhaltungen:
**DE - A - 2 038 543**

**CHEMICAL ABSTRACTS, Band 87, Nr. 24, 12. Dezember 1977, Seite 64, Nr. 185850q, Columbus, Ohio, USA**

(73) Patentinhaber: **Rehau Plastiks AG + Co, Rheniumhaus, D-8673 Rehau (DE)**

(72) Erfinder: **Rothemund, Karl, Am Rabenberg 20, D-8671 Schönwald (DE)**

EP 0 059 912 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es sind eine Anzahl von Gehörschutzelementen bekannt, die in den menschlichen Gehörgang zur Vermeidung oder Abschwächung gesundheitsgefährdender Lärmbelästigung eingeführt werden. Hierfür wird einfache Watte genauso verwendet wie solche Wattefasern, die mit wachsartigen Substanzen knetbarer Eigenschaften imprägniert sind.

Aus der Deutschen Offenlegungsschrift 2 251 774 ist ein Gehörschutzelement aus geschäumtem, polymerem Material bekannt, welches sich aufgrund der besonderen Schaumstruktur durch Druckanwendung komprimieren und im komprimierten Zustand in den Gehörgang einführen lässt. Aufgrund der langsamen Erholungsfähigkeit des komprimierten, geschäumten Polymeren stellt sich das Element innerhalb kurzer Frist im Gehörgang so weit zurück, dass sich der äussere Umfang des Elementes an die Oberfläche des Gehörgangs anlegt und auf diese Weise einen Abschluss schafft, der das Eindringen von gesundheitsschädlichem Lärm in das Innere des Gehörgangs vermindert bzw. verhindert. Aus dieser Vorveröffentlichung ergibt sich, dass jedes beliebige, flexible polymere Material, das geschäumt werden kann, zur Schaffung eines solchen Gehörschutz-Stopfens verwendet werden kann. Als wesentliches Kriterium wird dabei gesehen, dass die in der Vorveröffentlichung angegebenen Daten über den zur Komprimierung erforderlichen Druck und die Erholungsgeschwindigkeit des geschäumten Polymeren eingehalten werden.

Die Vorveröffentlichung verwendet Vinylchlorid-Mischungen, schäumbare Polyvinylchlorid-Plastisol-Mischungen und Polyvinylchlorid-Mischungen. Diese werden mit Weichmachern und Treibmitteln versetzt, die ein entsprechend geschäumtes, plattenförmiges Produkt ergeben, aus dem dann die einzelnen Gehörschutz-Stopfen ausgestanzt werden.

Als besonderer Vorteil wird von der Vorveröffentlichung angegeben, dass auf diese Weise komprimierbare, stopfenartige Gehörschutz-Elemente geschaffen werden, die aufgrund ihrer Kompressionsfähigkeit an jede Grösse und jede Form des menschlichen Gehörgangs anpassbar sind. Dieser Vorteil wird in der Vorveröffentlichung als so wesentlich angesehen, dass es als nicht möglich bezeichnet wird, einen Gehörschutz-Stopfen aus geformtem elastomerem Material herzustellen, der eine allseitig verwendbare Grösse und Form aufweist. Solche Materialien werden wegen ihrer hohen Elastizität als ungeeignet für die Herstellung von Gehörschutz-Stopfen bezeichnet. Insbesondere auch die rasche Rückstellung soll nach dieser Ansicht eine geeignete Einführung des Gehörschutz-Stopfens in den Gehörgang behindern.

Als Nachteil der bekannten Ausführungsform ist zu werten, dass in den verwendeten schäumbaren Polymeren, z.B. Polyvinylchlorid weich, auch Polyurethan usw., niedrig molekulare Weichmacher, Stabilisatoren oder sonstige Verarbeitungsrückstände enthalten sind, die aus dem fertigen Gehörschutz-Stopfen nicht entfernt werden können. Diese Bestandteile sind nicht entfernbar und können bei der Verwendung als Gehörschutz-Stopfen, d.h. nach dem Einsetzen des Stopfens in den menschlichen Gehörgang, zu Hautreizungen, Kontaktdermatitis oder sonstigen allergischen Hautreaktionen führen. Nachdem das schäumbare Polymere der Vorveröffentlichung zunächst in Plattenform gebracht und danach erst die eigentliche Stopfenform durch Ausschneiden, Ausstanzen oder ähnliche mechanische Arbeiten erzielt wird, ergibt sich auch der Nachteil, dass die Oberfläche dieser Stopfen zwangsläufig porös ist. Dadurch wird ein gewisser Scheuereffekt zwischen der Stopfenoberfläche und der Gehörgang-Oberfläche zwangsläufig erzielt, so dass die vogenannten negativen Hautbeeinflussungen dadurch noch unterstützt werden.

Als weiterer Nachteil ist die Anfälligkeit der verwendeten geschäumten Polymere gegen Befall von Mikroorganismen wie Pilze, Bakterien usw. zu nennen. Insbesondere bei Polyvinylchlorid weich ist diese Anfälligkeit bekannt, während bei der Verwendung von Polyurethan-Schäumen der Bakterienfrass eine besondere Rolle spielt. Bei Temperaturen, wie sie im menschlichen Gehörgang herrschen, haben darüber hinaus diese schädlichen Pilze, Bakterien usw. optimale Wachstumsbedingungen, was wiederum die entzündlichen Reaktionen im Gehörgang fördern kann. Dieser Effekt wird noch verstärkt durch die aus der Bearbeitung stammende poröse Oberfläche der bekannten Gehörschutz-Stopfen, die darüber hinaus ein wesentliches Element für die leichte Verschmutzung des Stopfens bei seinem Einsatz darstellt.

Aus der US-Patentschrift 4 160 449 ist ein Gehörschutz-Stopfen bekannt, dessen rückstellfähiges Stopfenmaterial aus Silikon-Schaumstoff besteht, welches aus hygienischen Gründen von einer dünnen Plastikfolie überzogen ist. Die Umhüllung mit Plastikfolie ist erforderlich, weil der aus geschäumtem Plattenmaterial ausgestanzte oder aus geschäumtem Stabmaterial abgeschnittene Stopfen zumindest an den Schnittstellen grobporig bzw. offenzellig ist und damit die nachteiligen Hautreizungen oder allergische Hautreaktionen herbeiführen kann.

Hier setzt die Erfindung ein, die es sich zur Aufgabe gestellt hat, die vorgenannten Nachteile zu vermeiden und ein Verfahren anzugeben, welches ohne zusätzliche Hilfsmittel einen physiologisch einwandfreien Gehörschutz-Stopfen erbringt. Erfindungsgemäss wird dazu vorgeschlagen, dass zunächst das Polysiloxan mit den Zuschlagstoffen versetzt und vorgeformt wird, dass anschliessend die Endausformung durch den Schäumvorgang erfolgt und das endgeformte Produkt vernetzt wird, und dass schliesslich das endgeformte Produkt einem abschliessenden Tempervorgang bei Temperaturen von wenigstens 140°C unterzogen wird.

Mit der Erfindung wird ein elastomeres Ausgangsmaterial verwendet, welches durch Zusätze von Treibmitteln und Vernetzungsmitteln besondere Eigenschaften enthält. Darüber hinaus können bei Bedarf entsprechende Füllstoffe in das Kunststoff-Ausgangsmaterial eingearbeitet werden.

Als Treibmittel können hierbei chemische Substanzen eingesetzt werden, die unter Einwirkung von Temperatur und Gasabspaltung zerfallen, wie z.B. Azodicarbonamid, Benzolsulfohydrazid, Diphenylsulfon-3,3 Disulfohydrazid, Diphenylenoxid-4,4 Disulfohydrazid, Trihydrazionotriazin, P-Toluensulfonylsemicarbazid, 5-Phenyltetrazol, Isatosäureanhydrid, Natriumhydrogencarbonat usw.

Es können aber auch physikalische Treibmittel, die unter Einwirkung von Wärme verdampfen und dabei expandieren, verwendet werden. Zur Vernetzung werden den Polysiloxanen radikalbildende Vernetzer, insbesondere Benzoylperoxid, Chlorbenzoylperoxid, Dichlorbenzoylperoxid, t-Butylperbenzoat, Dicumylperoxid oder Di-t-Butylperoxid beigefügt. Die Vernetzung kann jedoch auch durch Additionsvernetzung oder mittels Kondensationsreaktion unter Austritt von Wasser, Essigsäure oder Aminen bei Raumtemperatur oder in der Hitze erfolgen. Es können hierbei Ein- oder Mehrkomponentensysteme zur Anwendung kommen, wobei — insgesamt gesehen — die Vernetzung kontinuierlich oder diskontinuierlich durchgeführt werden kann.

Neben den genannten Vernetzungsarten kann die Vernetzungsreaktion selbstverständlich auch durch Zuführung energiereicher Strahlung erfolgen.

Als Füllstoffe kann das vorgeschlagene Polysiloxan Russe sowie anorganische Füllstoffe, besonders helle, inaktive oder halbaktive verstärkte Füllstoffe, enthalten. Diese können aus oxidischen oder salzartigen Verbindungen des Kalziums, Bariums, Magnesiums, Aluminiums, Siliciums, Titans, Eisens, Zinks oder Zirkoniums bestehen. Diese Füllstoffe sind vorteilhaft mit ihren Einzelpartikeln vollumfänglich von dem Polysiloxan eingeschlossen, wodurch optimale Oberflächeneigenschaften des Endproduktes erzielbar sind.

Eine typische Siloxan-Verbindung des hier verwendeten Typs wird durch nachfolgende Formel gekennzeichnet:

$$R_3Si - O\ (\text{-}\overset{\displaystyle R}{\underset{\displaystyle R}{Si}}\text{-O-}\overset{\displaystyle R}{\underset{\displaystyle R}{Si}}\text{-O})_n\ SiR_3$$

wobei R = organischer Rest, insbesondere Methyl-, Phenyl-, Vinyl-Gruppe, Si = Silicium und O = Sauerstoff bedeutet.

Die Verarbeitung dieses Grundpolymeren zu einem Gehörschutz-Stopfen ist ohne Vernetzung überhaupt nicht möglich. Nachdem im Stand der Technik die direkte Verwendung solcher vernetzten Polymeren für diesen Einsatzzweck wegen der negativen Eigenschaften — hohe Elastizität und rasche Rückstellung — abgelehnt wurde, verwendet die Erfindung spezielle Zuschlagstoffe zu den Grundpolymeren, eine spezielle Temperaturführung beim Vernetzungsprozess und dem anschliessenden Temperprozess mit dem Ziel, ein Endprodukt aus vernetztem Polysiloxan zu erzielen, welches aufgrund seiner Eigenschaften wie hohes Rückstellungsvermögen bei relativ langsamer Rückstellzeit bei geschlossener Oberfläche als Gehörschutz-Stopfen direkt einsetzbar ist.

Eine erfindungsgemäss einsetzbare Rezeptur kann dabei wie folgt aufgebaut sein:

70 Gewichtsteile Dimethylpolysiloxan
30 Gewichtsteile pyrogengewonnene Kieselsäure
3 Gewichtsteile Azodicarbonamid
1 Gewichtsteil Dichlorbenzoylperoxid

Nach dem vorgegebenen Rezepturbeispiel wurden die Bestandteile innig miteinander vermischt und einem Formgebungsprozess zugeführt. Danach wurde das vorgeformte Zwischenprodukt bei ca. 210°C für die Dauer von 5 Minuten erhitzt, dabei endgeschäumt und gleichzeitig vernetzt. Im Anschluss daran wurde das vernetzte Endprodukt bei ca. 180°C für die Dauer von 6 Stunden getempert. Auf diese Weise wurde ein physiologisch unbedenkliches Endprodukt erhalten, welches zum Einsatz als Gehörschutz-Stopfen geeignet war.

Das Endprodukt zeichnet sich durch ein sehr gutes Rückstellvermögen aus. Ferner muss es als überraschend gewertet werden, dass trotz des verwendeten Werkstoffes eine langsame Rückstellzeit erzielt werden konnte, die das Produkt u.a. erst zum Einsatz für diesen Zweck geeignet macht. Die besonderen Eigenschaften des Endproduktes nach der Erfindung wurden am Gleichgewichtsdruck und an der Rückstellzeit eines erfindungsgemässen Gehörschutz-Stopfens überprüft.

Die Prüfung des Gleichgewichtsdruckes erfolgte durch Umformung des zylindrischen Prüfkörpers in radialer Richtung, wobei die aufgegebenen Werte als Mittelwerte verschiedener Einzelmessungen gesehen werden müssen. Verwendet wurde ein Prüfkörper mit einem Durchmesser von 11,1 mm und einer Länge von 16,3 mm. Die Verformungsgeschwindigkeit betrug 20 mm/Min., die Verformungskraft lag bei 70%. Die dabei erzielten Mittelwerte ergaben sich wie folgt:

| | |
|---|---|
| Verformung | 20,8 N |
| Gleichgewichtsdruck | 0,062 $N/mm^2$ |
| Hysterese | 56,8% |

Die Rückstellzeit des Prüfkörpers wurde wie folgt ermittelt: Der Prüfkörper wurde mit einem Gewicht von 5 kg für die Dauer von 30 Sekunden in radialer Richtung belastet. Für die Rückstellung auf 90% des ursprünglichen Durchmessers benötigte der Prüfkörper 49 Sekunden.

Durch die Verwendung des beschriebenen Polysiloxans ist die besondere Temperaturbeständigkeit der gewonnenen Produkte vorgegeben. Erfindungsgemäss kann die Verschäumungs- und die Vernetzungstemperatur des Materials unter 250°C, vorzugsweise zwischen 90°C und 160°C liegen. Schon allein dadurch wird die Verwendung der aus der Deutschen Offenlegungsschrift 2 251 774 bekannten schäumfähigen Polymere ausgeschlossen. Die dort verwendeten thermoplastischen Polymere haben ihre besonderen Eigenschaften durch die reversible Formgebung, während die vernetzten Polymere der Erfindung diese Reversibilität nicht besitzen, sondern nach Durchführung des erfindungsgemässen Verfahrens ihren Endzustand aufweisen. Diese Reversibilität ist selbst dann nicht gegeben, wenn erfindungsgemäss das endgeformte Produkt einem Tempervorgang von wenigstens 140°C unterworfen wird. Darüber hinaus führt die Temperaturbeständigkeit des Endproduktes nach der Erfindung dazu, dass diese Produkte durch Auskochen, durch Behandlung mit Heissdampf, Heissluft usw. mehrfach sterilisiert werden können. Auch daraus wird deutlich, dass die nach dem Stand der Technik bekannten, weich eingestellten, schäumfähigen Polymere für das Verfahren nach der Erfindung nicht geeignet sind. Dort bleiben vielmehr die für die weiche Einstellung des Polymeren erforderlichen Weichmacher und sonstigen Zuschlagstoffe mit ihren schädlichen Rückständen im Endprodukt erhalten, während ähnlich schädliche Rückstände durch den Vernetzungs- und den nachfolgenden Tempervorgang bei der Erfindung aus dem Endprodukt entfernt werden. Später bei der Benutzung aufgenommene Fremdstoffe können durch die Sterilisation beseitigt werden.

Nach dem erfindungsgemässen Verfahren wird das Polysiloxan mit den Zuschlagstoffen zunächst vorgeformt. Hierbei kann bereits die gewünschte Endform berücksichtigt werden, was beispielsweise durch die Verformung mittels Extrusion ein rundes oder ovales, strangförmiges Vorprodukt erbringen kann.

Es liegt im Rahmen der Erfindung, dass ein solches Vorprodukt anschliessend durch beispielsweise Wärmezuführung endgeschäumt wird. Dabei kann die glatte und porenfreie Oberfläche des Endproduktes erzielt werden. Gleichzeitig kann durch diese Wärmezuführung die Vernetzung erfolgen. Bei einer Vernetzung mittels energiereicher Strahlen wird man — und auch das liegt im Rahmen der Erfindung — das Vorschäumen und das Endschäumen des Produktes zunächst durchführen und dann dem Endprodukt energiereiche Strahlung zuführen. Man kann aber in diesem Fall auch auf das Vorschäumen verzichten und gleich den Schäumvorgang so ablaufen lassen, dass sofort die Endform des Produktes erzielt wird.

Gegenüber dem bekannten Stand der Technik bietet die Erfindung den Vorteil, dass ein Gehörschutz-Stopfen mit einer porenfreien, geschmeidig-weichen Aussenhaut erzielt wird. Nach dem Stand der Technik war dies bisher nicht möglich, wie sich aus dem Deutschen Gebrauchsmuster 1 905 618 ergibt, in dem ein Gehörschutz-Stopfen aus einem Schaumstoffkern beschrieben ist, wobei dieser Schaumstoffkern mit einer elastischen Gummihaut überzogen wurde.

Wenn das vorgeformte Produkt gemäss dem Verfahren der Erfindung vor der Endverschäumung und Vernetzung in Einzelabschnitte unterteilt wird, kann man auch erreichen, dass die Stirnflächen des Gehörschutz-Stopfens von einer gleichartigen, porenfreien, geschmeidig-weichen Oberflächenstruktur sind wie die Umfangsflächen des Stopfens. Die Einzelabschnitte können ausserdem vorteilhaft durch Anwendung eines Pressdruckes zusätzlich geformt und erst dann anschliessend geschäumt und vernetzt werden. Dies gibt die Möglichkeit, individuell gestaltete Gehörschutz-Stopfen mit den Vorteilen der Erfindung auszustatten.

Der Schäumvorgang kann unter Anwendung von Wärme durchgeführt werden, wobei diese Wärme gleichzeitig die Vernetzungswärme darstellen kann. In diesem Fall kann ein einstufiges Verfahren durchgeführt werden, mit welchem nach der Verformung des Produktes in einem einzigen Arbeitsgang ein endgeformtes und vernetztes Endprodukt erhalten wird. Das erfindungsgemässe Verfahren kann jedoch auch derart ablaufen, dass zunächst das Endprodukt geschäumt und dann anschliessend vernetzt wird, wobei beispielsweise zur Vernetzung energiereiche Strahlung verwendet wird.

Ein wesentliches Merkmal des erfindungsgemässen Verfahrens ist der abschliessende Tempervorgang, der bei den weich eingestellten Polymeren des Standes der Technik nicht durchgeführt werden kann. Mit diesem Tempervorgang, der bei Temperaturen von wenigstens 140°C und einstellbarem Zeitablauf erfolgt, wird die Entfernung von Vernetzer- und Treibmittelrückständen bewirkt. Bei diesen Rückständen handelt sich sich z.B. um 1,3-Dichlor-benzol, 2,4-Dichlorbenzaldehyd, 2,4-Dichlorbenzoesäure oder Benzol, Benzaldehyd, Benzoesäure; oder 1,1,3-Trimethylcyclohexanon, 1,1,3-Trimethylcyclohexanol; oder t-Butanol, Isobutan, t-Amylalkohol, 2-Methylbutan oder Chlorbenzol, 4-Chlorbenzaldehyd, 4-Chlorbenzoesäure usw. Derartige niedermolekulare Anteile werden durch die Temperbehandlung oberhalb 140°C vollständig aus dem erfindungsgemässen Gehörschutz-Stopfen entfernt.

Die mit diesem Verfahren hergestellten Gehörschutz-Stopfen mit porenfreier, geschmeidig-weicher Aussenhaut werden — wie beim bekannten Stand der Technik — nach einem Walkvorgang, durch welchen der Stopfen komprimiert wird, in den menschlichen Gehörgang eingeführt. Durch die besondere Oberflächenform und -ausgestaltung ergibt sich bei der Rückstellung ein deutlich verringerter Druck auf den Gehörgang, wodurch das Tragen solcher Gehörschutz-Stopfen angenehmer wird.

Durch die Verwendung eines temperfähigen Ausgangsmaterials und durch die Temperung des Produktes in der Endform selbst wird zusätzlich zu der auf den Träger solcher Stopfen wirkenden angenehmeren Anwendungsform durch die Austreibung der niedermolekularen Rückstände eine physiologisch einwandfreie Eigenschaft des Endproduktes erzielt. Hautreizungen, Kontaktdermatitis, Allergien, starke Entfettung und Versprödung der Haut usw. durch die Rückstände von Isocyanaten, Polyolen, Halogen-Kohlenwasserstoffen und Aktivatoren sind bei der Anwendung eines nach dem erfindungsgemässen Verfahren unter Verwendung des erfindungsgemässen Polysiloxans hergestellten Gehörschutz-Stopfens ausgeschlossen.

**Patentansprüche**

1. Verfahren zur Herstellung eines Gehörschutz-Stopfens aus Polysiloxan-Schaumstoff, dadurch gekennzeichnet, dass zunächst das Polysiloxan mit Zuschlagstoffen versetzt und vorgeformt wird, dass anschliessend die Endausformung durch den Schäumvorgang erfolgt und das endgeformte Produkt vernetzt wird, und dass schliesslich das endgeformte Produkt einem abschliessenden Tempervorgang bei Temperaturen von wenigstens 140°C unterzogen wird.

2. Verfahren nach Anspruch 1, dadurch gekennzeichnet, dass das vorgeformte Produkt vor der Verschäumung und Vernetzung in Einzelabschnitte unterteilt wird.

3. Verfahren nach Ansprüchen 1 und 2, dadurch gekennzeichnet, dass die Einzelabschnitte durch Anwendung eines Pressdruckes zusätzlich verformt und anschliessend geschäumt und vernetzt werden.

4. Verfahren nach Anspruch 1, dadurch gekennzeichnet, dass das Produkt nach der Verschäumung und Vernetzung in Einzelabschnitte unterteilt wird.

5. Verfahren nach Anspruch 1, dadurch gekennzeichnet, dass der Schäumvorgang unter Anwendung von Wärme durchgeführt wird, die gleichzeitig die Vernetzungswärme ist.

6. Verfahren nach Anspruch 1, dadurch gekennzeichnet, dass der Schäumvorgang unter Anwendung von Wärme durchgeführt und anschliessend das endgeschäumte Produkt vernetzt wird.

7. Verfahren nach Anspruch 6, dadurch gekennzeichnet, dass die Vernetzung durch energiereiche Strahlung erfolgt.

8. Verfahren nach Ansprüchen 1 bis 6, dadurch gekennzeichnet, dass die Verschäumungswärme und die Vernetzungswärme unter 250°C, vorzugsweise zwischen 90°C und 160°C, liegt.

**Claims**

1. A process for manufacturing ear plugs in polysiloxane foam wherein the polysiloxane is first mixed with additives and preformed, subsequently final forming is effected by a foaming process and the end product is cross-linked and finally the end product is subjected to a final tempering process at temperatures of minimum 140°C.

2. A process according to claim 1 wherein the preformed product is divided in individual sections prior to foaming and cross-linking.

3. A process according to claim 1 and 2 wherein the individual sections are additionally shaped by applying pressure and subsequently foamed and cross-linked.

4. A process according to claim 1 wherein the product is divided in individual sections after foaming and cross-linking.

5. A process according to claim 1 wherein foaming is effected by applying heat which is at the same time the heat for cross-linking.

6. A process according to claim 1 wherein foaming is effected by applying heat and the finally foamed product is subsequently cross-linked.

7. A process according to claim 6 wherein cross-linking is effected by high-energy radiation.

8. A process according to claims 1 to 6 wherein foaming heat and cross-linking heat are below 250°C and preferably between 90°C and 160°C.

**Revendications**

1. Procédé de fabrication de tampons auriculaires en polysiloxane cellulaire, caractérisé en ce que le polysiloxane est tout d'abord mélangé à des additifs et préformé, que le façonnage final est effectué par le procédé moussant et que le produit, une fois formé, est réticulé et que pour finir le produit une fois formé est soumis à un procédé d'étuvation après cuisson à des températures d'au moins 140°C.

2. Procédé suivant la revendication 1, caractérisé en ce que le produit préformé est divisé en morceaux avant la transformation en mousse et la réticulation.

3. Procédé suivant les revendications 1 et 2, caractérisé en ce que les morceaux sont déformés encore plus par l'utilisation d'une pression, puis moussés et réticulés.

4. Procédé suivant la revendication 1, caractérisé en ce que le produit est divisé en morceaux après la transformation en mousse et la réticulation.

5. Procédé suivant la revendication 1, caractérisé en ce que le procédé moussant est effectué en utilisant de la chaleur qui est en même temps la chaleur de réticulation.

6. Procédé suivant la revendication 1, caractérisé en ce que le procédé moussant est effectué en utilisant de la chaleur et qu'ensuite le produit transformé en mousse est réticulé.

7. Procédé suivant la revendication 6, caractérisé en ce que la réticulation est effectuée par radiation de haute énergie.

8. Procédé suivant les revendications 1 à 6, caractérisé en ce que la chaleur de transformation en mousse et la chaleur de réticulation sont inférieures à 250°C, de préférence entre 90°C et 160°C.